**Europäisches Patentamt**

**European Patent Office**

**Office européen des brevets**

(19)

(11) Veröffentlichungsnummer: **0 012 299**
**B1**

(12) # EUROPÄISCHE PATENTSCHRIFT

(45) Veröffentlichungstag der Patentschrift:
**12.05.82**

(51) Int. Cl.³: **C 07 C 59/105,** C 07 C 51/43,
C 07 C 51/353, C 07 C 51/16

(21) Anmeldenummer: **79104811.9**

(22) Anmeldetag: **01.12.79**

(54) **Lithiumribonat, Lithiumarabonat sowie Verfahren zur Herstellung und Reindarstellung dieser Salze.**

(30) Priorität: **06.12.78 DE 2852720**

(43) Veröffentlichungstag der Anmeldung:
**25.06.80 Patentblatt 80/13**

(45) Bekanntmachung des Hinweises auf die Patenterteilung:
**12.05.82 Patentblatt 82/19**

(84) Benannte Vertragsstaaten:
**CH DE FR GB**

(56) Entgegenhaltungen:
**DE-A1-2 628 056**
**DE-C-1 148 991**

(73) Patentinhaber: **BASF Aktiengesellschaft,
Carl-Bosch-Strasse 38, D-6700 Ludwigshafen (DE)**

(72) Erfinder: **Schmidt, Wolfram, Dr. Dipl.-Chem., Im
Rosengarten Ost 8, D-6701 Friedelsheim (DE)**
Erfinder: **Paust, Joachim, Dr. Dipl.-Chem., Ringstrasse 3,
D-6701 Neuhofen (DE)**

## Lithiumribonat, Lithiumarabonat sowie Verfahren
## zur Herstellung und Reindarstellung dieser Salze

Die vorliegende Erfindung betrifft Lithiumribonat (Ia) und Lithiumarabonat (Ib) sowie Verfahren zur Herstellung und Reindarstellung dieser Salze.

Wie allgemein bekannt ist, haben Ribonsäure bzw. deren Salze als Zwischenprodukte für die Synthese von Riboflavin (Vitamin B$_2$) große Bedeutung. Man erhält die Ribonate, indem man Glucose in wäßriger Natron- oder Kalilauge mit Sauerstoff zu einem Gemisch aus Na- bzw. K-Arabonat und Na- bzw. K-Formiat oxidiert, aus dieser Lösung das Arabonat durch Zugabe eines mit Wasser mischbaren Lösungsmittels isoliert und das Arabonat anschließend durch Erhitzen in wäßriger Lösung auf 130 bis 140° C epimerisiert.

Da diese Epimerisierung stets nur bis zu einem Gleichgewicht von etwa 70% Arabonat mit 30% Ribonat führt, ist eine fraktionierte Kristallisation von Arabonat und Ribonat erforderlich. Hierzu eignen sich die Natrium- und Kaliumsalze jedoch nicht, da diese extrem wasserlöslich sind und sich außerdem nicht genügend in ihrer Löslichkeit unterscheiden. Infolgedessen versetzt man im allgemeinen die Ia und Ib enthaltenden wäßrigen Lösungen mit Calciumchlorid, worauf nach dem Abkühlen das Calciumarabonat bevorzugt auskristallisiert (vgl. japanische Patentveröffentlichung 4 525/1955). Eine Variante dieser Verfahrensweise besteht darin, das Na- bzw. K-Arabonat zunächst in das Ca-Arabonat zu überführen, dieses abzutrennen und die Epimerisierung anschließend vorzunehmen (vgl. US 2 438 882). Bei der fraktionierten Kristallisation der genannten epimeren Calciumsalze kann man ein Ribonat etwa 80%iger Reinheit erhalten.

Zur Erzielung höherer Reinheiten überführt man die Ca-Salze in andere Salze, die durch weitere Kristallisation gereinigt werden können. Bekannt sind Trennungen über die Zink-, Cadmium- oder Quecksilbersalze, die aber aufgrund der Giftigkeit der Metallsalze und der hierdurch bedingten Abwasserprobleme für ein technisches Verfahren ungeeignet sind.

Die ebenfalls für diesen Zweck verwendeten Eisensalze sind zwar nicht giftig, haben aber andere entscheidende Nachteile. So bedarf es beispielsweise zur Überführung der Calciumsalze in die Eisensalze eines 3- bis 5stündigen Erhitzens auf 80 bis 100° C, was technisch unattraktiv ist. Außerdem gelingt die Überführung des Eisenribonats in Ribonolacton nur mit Ausbeuten von etwa 80 bis 85%, was für eine technische Synthese unbefriedigend ist.

Der Erfindung lag daher die Aufgabe zugrunde, Ribonat und Arabonat auf einfachere Weise voneinander zu trennen als bisher.

Es wurden Lithiumribonat (Ia) und Lithiumarabonat (Ib) als neue Verbindungen gefunden, mit deren Hilfe die erfindungsgemäße Aufgabe auf elegante Weise gelöst werden konnte.

Es wurde nämlich überraschenderweise gefunden, daß sich in wäßriger Lösung vorliegende Gemische aus Ia und Ib mit hervorragendem Erfolg durch fraktionierte Kristallisation in ihre Komponenten trennen lassen, wobei es möglich ist, das gewünschte und hierbei zuerst kristallisierende, schwerer lösliche Ia in sehr reiner Form abzutrennen. Die verbleibende, hauptsächlich Ib enthaltende Lösung kann ohne großen Aufwand erneut epimerisiert werden, woran sich dann wieder die erfindungsgemäße Kristallisation anschließen kann.

Man erhält die wäßrigen Ia und Ib enthaltenden Lösungen in an sich bekannter Weise durch Epimerisierung von Lithiumarabonat durch Erhitzen in wäßriger Lösung auf 130 bis 140° C ggf. in Gegenwart von katalytischen Mengen einer Base wie Pyridin, Picolin, Dimethylamin oder anorganischen basischen Ionenaustauschern in neutralem oder alkalischem Milieu.

Das Lithiumarabonat ist ähnlich wie die übrigen Alkaliarabonate leicht zugänglich. Man erhält es dadurch, daß man Glucose in wäßriger lithiumalkalischer Lösung in an sich bekannter Weise mit Sauerstoff oxidiert und aus der hierbei erhaltenen Lösung von Lithiumarabonat und Lithiumformiat das Lithiumarabonat durch Zugabe eines mit Wasser mischbaren Lösungsmittels in kristalliner Form ausfällt.

Eine weitere Möglichkeit zu den Li-Salzen zu gelangen besteht darin, eine wäßrige Lösung der Kalium-Salze mit einer Mineralsäure, wie Schwefelsäure, anzusäuern, das Kaliumsalz der Mineralsäure mit Hilfe eines wasserlöslichen organischen Lösungsmittels, z. B. mit Äthanol, auszufällen und die verbleibende Lösung mit Lithiumhydroxid zu versetzen. Ferner kann man das Kalium im Kaliumarabonat an einem mit Lithium beladenen Kationenaustauscher gegen Lithium austauschen.

Gegenstand der Erfindung ist neben den neuen Lithiumsalzen Ia und Ib ein Verfahren zur Reindarstellung von Lithiumribonat, das dadurch gekennzeichnet ist, daß man eine wäßrige Lithiumribonat- und Lithiumarabonat enthaltende Lösung mit 40 bis 900, vorzugsweise 60 bis 300 Gew.-%, bezogen auf die Menge des Wassers, eines wasserlöslichen nichtionogenen oder praktisch nichtionogenen organischen Lösungsmittels versetzt und anschließend hieraus bei 0 bis 20° C das Lithiumribonat auskristallisieren läßt. Mit diesem Verfahren ist es möglich, das Lithiumribonat durch einmalige Kristallisation in bis zu 95%iger Reinheit zu gewinnen. Das Lithiumarabonat kann — wenn es zur Herstellung von Lithiumribonat verwendet werden soll — gleich in Form der durch Abdestillieren des nichtionogenen Lösungsmittels erhaltenen wäßrigen Lösung erneut einer Epimerisierung zugeführt werden.

Gegenstand der Erfindung ist weiterhin ein Verfahren zur Herstellung der wäßrigen Lösung von Gemischen aus Lithiumribonat und Lithiumarabonat, das dadurch gekennzeichnet ist, daß man Lithiumarabonat in an sich bekannter Weise durch Erhitzen in wäßriger Lösung auf 130 bis 140°C zu einem Gemisch aus Lithiumribonat und -arabonat epimerisiert.

Die wäßrigen Ia und Ib enthaltenden Lösungen stellt man für die fraktionierte Kristallisation zweckmäßigerweise auf eine Konzentration von 10 bis 60, vorzugsweise 20 bis 40 Gew.-% Gesamtgehalt an epimeren Li-Salzen. Hiervon entfallen, bezogen auf den Gesamtgehalt (=100%) im allgemeinen etwa 25 bis 30% auf das Ribonat und der Rest auf das Arabonat. Man kann das erfindungsgemäße Verfahren jedoch beispielsweise auch zur Feinreinigung der bei der fraktionierten Kristallisation der epimeren Ca-Salze anfallenden etwa 70 bis 80% Ribonat und 20 bis 30% Arabonat enthaltenden Mutterlaugen verwenden (vgl. Beispiele 5 und 6).

Die Kristallisation kann man je nach Konzentration der Lithiumsalze in der wäßrigen Lösung und je nach Art und Menge des verwendeten Lösungsmittels bei Temperaturen von 0—90°C durchführen. Bevorzugt werden Temperaturen von Raumtemperatur bis 70°C, insbesondere 40—70°C.

Die Trennung der Epimeren über die Li-Salze bietet gegenüber der Trennung über die Ca-Salze den besonderen Vorteil, daß das Lithiumribonat Ia schwerer löslich ist als das Lithiumarabonat Ib. Ist nämlich — wie bei den Calciumsalzen — das Arabonat schwerer löslich, so muß man dieses auf umständliche Weise weitgehend auskristallisieren, damit nicht zu viel Arabonat neben dem erwünschten Ribonat in der Mutterlauge verbleibt. Trotzdem kann man auf diese Weise nur Reinheiten an Ribonat von ungefähr 70—80% erzielen. Ist umgekehrt — wie im Falle der Lithiumsalze — das Ribonat schwerer löslich, so kann man sich mit einer Kristallisation von etwa 30 bis 60% des Ribonats begnügen, wobei man Reinheiten an Lithiumribonat von 95 bis 98% erzielt.

Als definitionsgemäße organische Lösungsmittel eignen sich insbesondere $C_1$- bis $C_4$-Alkanole wie Methanol, Äthanol, Isopropanol und n-Butanol; cycloaliphatische Äther wie Dioxan sowie mit Methanol oder Äthanol partiell verätherte Alkandiole mit 2 oder 3 C-Atomen. Besonders gut geeignet sind Methanol sowie der Monomethyl- und Monoäthyläther des Äthylenglykols. Auch Mischungen dieser Lösungsmittel können verwendet werden.

Zweckmäßigerweise verwendet man die Lösungsmittel in Mengen von 40 bis 900, vorzugsweise 60 bis 300 Gew.-%, bezogen auf das Wasser.

Es empfiehlt sich, die Kristallisation jeweils mit ein paar Lithiumribonat-Kristallen zu initiieren. Reines Lithiumribonat kristallisiert aus Methanol in Form farbloser Kristalle und hat einen Schmelzpunkt (unter Zersetzung) von 201°C. Reines Lithiumarabonat kristallisiert aus Methanol in Form farbloser Kristalle, die 1 Mol Kristallwasser enthalten. Die Verbindung schmilzt unter Zersetzung bei 167—169°C.

Die Bedeutung des Lithiumarabonats liegt insbesondere darin, daß es durch Epimerisierung in der für den Kalium- und Natriumarabonat bekannten Weise jeweils bis zur Gleichgewichtseinstellung (etwa 70% Arabonat und 30% Ribonat) in das Lithiumribonat überführt werden kann, welches auf die erfindungsgemäße Weise auf sehr einfache Weise und in sehr reiner Form von dem nicht epimerisierten Lithiumarabonat abgetrennt werden kann.

Reines Lithiumribonat ist eine wichtige Vorstufe für die Synthese von Riboflavin, indem es in an sich bekannter Weise mit Säuren oder einem sauren Ionenaustauscher in das Ribonolacton überführt wird. Die erfindungsgemäßen Verbindungen ermöglichen eine besonders wirtschaftliche und elegante Trennung von Ribonat und Arabonat und damit eine wesentlich vereinfachte Gesamtsynthese des Riboflavins. Das Schema dieser Synthese unter Verwendung der erfindungsgemäßen Verbindungen sowie der erfindungsgemäßen Verfahren ist nachstehend wiedergegeben.

$$\text{Glucose} \xrightarrow[\text{Wasser}]{\text{O}_2;\ \text{LiOH}} \text{Li-Arabonat} + \text{HCOOLi} \xrightarrow{\text{wassermischbare Lösungsmittel}} \text{Li-Arabonat}$$

$$\text{Li-Arabonat} + \text{Wasser} \xrightarrow{130-140^\circ\text{C}} \text{Li-Arabonat} + \text{Li-Ribonat}$$

fraktionierte Kristallisation

Lösung von
Li-Arabonat
+
wenig Li-Ribonat

Li-Ribonat

$\downarrow$ Säure

Ribonolacton

Riboflavin

## Beispiel 1

### a) Herstellung von Lithiumarabonat durch Oxydation von Glucose

Eine Lösung von 65 g Glucose und 20 g LiOH in 650 ml Wasser wurde unter einem Druck von 20 bar Sauerstoff 6 Stunden lang auf Temperaturen von 45 bis 50°C erhitzt. Anschließend wurden aus dem Reaktionsgemisch etwa 560 ml Wasser abdestilliert und die Restlösung mit 210 ml Methanol versetzt. Es kristallisierten 50 g Lithiumarabonat aus. Die Ausbeute beträgt somit 90%.
Fp. = 167 − 169° C (unter Zersetzung).

### b) Herstellung von Lithiumarabonat aus Kaliumarabonat

Eine Lösung aus 41 g Kaliumarabonat in 35 ml Wasser wurde mit konz. Schwefelsäure versetzt bis die Lösung einen pH-Wert von etwa 2 aufwies. Anschließend wurden 120 ml Dioxan zugefügt, worauf 17 g $K_2SO_4$ kristallin ausfielen. Nach Abfiltrieren des $K_2SO_4$ versetzte man das Filtrat in der Wärme mit einer Lösung von 5 g LiOH in 10 ml Wasser. Nach Zugabe von weiteren 80 ml Dioxan kristallisierte Lithiumarabonat in etwa 95%iger Ausbeute aus.

## Beispiel 2

Eine Lösung aus 70 g Li-Arabonat, 30 g Li-Ribonat und 180 g Wasser, wie sie bei der Epimerisierung von 100 g Li-Arabonat anfiel, wurde bei 90°C mit 720 g Dioxan versetzt und bei 60°C mit Hilfe einiger Li-Ribonatkristalle der fraktionierten Kristallisation unterworfen. Nach 1 Stunde wurden die Kristalle bei 60°C abfiltriert. Die Ausbeute an Li-Ribonat 95%iger Reinheit betrug 15,5 g ( = 50%, bezogen auf eingesetztes Li-Ribonat).

## Beispiel 3

Eine Lösung aus 30 g Li-Ribonat, 70 g Li-Arabonat und 400 g Wasser, wie sie bei der Epimerisierung von 100 g Li-Arabonat durch Erhitzen der wäßrigen Lösung auf 130 bis 140°C, Entfärben mit Aktivkohle und anschließendem Einengen anfiel, wurde bei 30°C mit 400 ml Methanol versetzt und auf 0°C abgekühlt. Hierbei kristallisierten nach Animpfen mit einigen Li-Ribonatkristallen im Laufe von 12 Stunden 13,5 g Li-Ribonat von 94%iger Reinheit aus. Dies entspricht einer Ausbeute von 45%, bezogen auf in die Kristallisation eingesetztes Li-Ribonat.
Schmelzpunkt (unter Zersetzung) 201° C.

### Beispiel 4

Eine Lösung aus 30 g Li-Ribonat, 70 g Li-Arabonat und 250 g Wasser, wie sie bei der Epimerisierung von Li-Arabonat durch Erhitzen der wäßrigen Lösung auf 130 bis 140°C, Entfärben mit Aktivkohle und anschließendem Einengen der Lösung erhalten wurde, wurde bei 90°C mit 670 g (700 ml) Äthylenglykolmonomethyläther versetzt und bei 60°C der fraktionierten Kristallisation unterworfen. Nach Animpfen mit Li-Ribonat kristallisierten im Laufe von 1 Stunde 15 g (=50%, bezogen auf eingesetztes Li-Ribonat) Li-Ribonat 95%iger Reinheit aus.

### Beispiel 5

Eine Lösung aus 70 g Li-Ribonat, 30 g Li-Arabonat und 180 g Wasser, wurde bei 90°C mit 307 g (320 ml) Äthylenglykolmonomethyläther versetzt und eine Stunde lang bei 60°C gehalten. Hierbei fielen 61 g (=87%) Lithiumribonat von 95%iger Reinheit an.

Bei der Ausgangslösung handelte es sich um die Mutterlauge wie sie bei der Abtrennung von kristallinem Ca-Arabonat aus der bei der Epimerisierung von Ca-Arabonat anfallenden Lösung erhalten wird.

Aus dieser Lösung wurde das Calcium mit Schwefelsäure als Calciumsulfat ausgefüllt, wobei die verbleibende Lösung der epimeren Säuren mit Lithiumhydroxid in die entsprechende Lösung der Lithiumsalze überführt und eingeengt wurde.

### Beispiel 6

Eine Lösung aus 70 g Li-Ribonat, 30 g Li-Arabonat und 180 g Wasser, die analog der im Beispiel 5 beschriebenen Ausgangslösung durch Epimerisieren von Calciumarabonat, Abtrennen von kristallinem Calciumarabonat und anschließendes Überführen der Calciumsalze der erhaltenen Mutterlauge in die Lithiumsalze erhalten wurde, wurde bei 30°C mit 330 g Methanol versetzt und die Mischung bei 0°C 1 Stunde sich selbst überlassen. Hierbei kristallisierten 62 g (=88%) Lithiumribonat von 98%iger Reinheit aus.

## Patentansprüche

1. Lithiumribonat.
2. Lithiumarabonat.
3. Verfahren zur Herstellung von Lithiumarabonat, dadurch gekennzeichnet, daß man Glucose in wäßriger lithiumalkalischer Lösung in an sich bekannter Weise mit Sauerstoff oxidiert und aus der hierbei erhaltenen Lösung von Lithiumarabonat und Lithiumformiat das Lithiumarabonat durch Zugabe eines mit Wasser mischbaren Lösungsmittels in kristalliner Form ausfällt.
4. Verfahren zur Herstellung der wäßrigen Lösung von Gemischen aus Lithiumribonat und Lithiumarabonat, dadurch gekennzeichnet, daß man Lithiumarabonat in an sich bekannter Weise durch Erhitzen in wäßriger Lösung auf 130 bis 140°C zu einem Gemisch aus Lithiumribonat und -arabonat epimerisiert.
5. Verfahren zur Reindarstellung von Lithiumribonat, dadurch gekennzeichnet, daß man eine wäßrige Lithiumribonat und -arabonat enthaltende Lösung mit 40 bis 900 Gew.-%, bezogen auf die Menge des Wassers, eines wasserlöslichen, nichtionogenen oder praktisch nichtionogenen organischen Lösungsmittels versetzt und anschließend hieraus bei 0 bis 80°C das Lithiumribonat auskristallisieren läßt.

## Claims

1. Lithium ribonate.
2. Lithium arabonate.
3. A process for the preparation of lithium arabonate, characterized in that glucose is oxidized in an aqueous lithium hydroxide solution by means of oxygen, in the conventional manner, and the lithium arabonate is precipitated in a crystalline form from the solution obtained, which contains lithium arabonate and lithium formate, by adding a water-miscible solvent.
4. A process for the preparation of the aqueous solution of mixtures of lithium ribonate and lithium arabonate, characterized in that lithium arabonate is epimerized in the conventional manner by heating an aqueous solution thereof at 130 to 140°C, to form a mixture of lithium ribonate and lithium arabonate.
5. A process for the preparation of pure lithium ribonate, characterized in that an aqueous solution, containing lithium ribonate and lithium arabonate, is mixed with from 40 to 900% by weight, based on the amount of water, of a water-soluble, non-ionic or virtually non-ionic, organic solvent and the lithium ribonate is then allowed to crystallize from the mixture at from 0 to 80°C.

## Revendications

1. Le ribonate de lithium.

2. L'arabonate de lithium.

3. Procédé de préparation de l'arabonate de lithium, caractérisé en ce que l'on oxyde le glucose en solution aqueuse alcaline lithinée de manière connue en soi par l'oxygène et, à partir de la solution d'arabonate de lithium et de formiate de lithium ainsi obtenue, on précipite l'arabonate de lithium à l'état cristallisé par addition d'un solvant miscible à l'eau.

4. Procédé de préparation de la solution aqueuse de mélanges de ribonate de lithium et d'arabonate de lithium, caractérisé en ce que l'on soumet l'arabonate de lithium à épimérisation en un mélange de ribonate de lithium et d'arabonate de lithium, de manière connue en soi, par chauffage en solution aqueuse à une température de 130 à 140°C.

5. Procédé pour préparer le ribonate de lithium à l'état pur caractérisé en ce que, à une solution aqueuse contenant du ribonate et de l'arabonate de lithium on ajoute de 40 à 900% en poids, par rapport à la quantité de l'eau, d'un solvant organique hydrosoluble non ionique ou pratiquement non ionique et on laisse ensuite cristalliser le ribonate de lithium de la solution à une température de 0 à 80°C.